# DEMANDE DE BREVET EUROPEEN

(11) **EP 0 976 396 A1**
(43) Date de publication de la demande: **02.02.2000**
(21) Numéro de dépôt: 99401579.0
(22) Date de dépôt: 24.06.1999
(51) Int. Cl.: A61K 9/70, A61K 47/36, A61K 7/00

(54) **Patch cosmétique ou pharmaceutique et son conditionnement**

(30) Priorité: 30.07.1998 FR 9809795
(71) Demandeur: L'OREAL, 75008 Paris (FR)
(72) Inventeur: Gueret, Jean-Louis H., 75016 Paris (FR)
(74) Mandataire: Boulard, Denis

(57) **Abrégé**

La présente demande concerne un ensemble (1) comportant une coque (2) définissant au moins un compartiment (3) présentant un fond (4) et dont un bord délimite, dans une face opposée audit fond, une première ouverture (5), obturée par un premier élément de fermeture amovible (6), ledit compartiment (3) contenant de manière amovible un patch à la forme du compartiment, et constitué d'une matrice gélifiée obtenue par coulage dans ledit compartiment (3) d'une composition (P) contenant au moins un hydrocolloïde dans une phase aqueuse et au moins un composé actif.

## Description

La présente invention a trait à un ensemble formé d'un patch, cosmétique, pharmaceutique, ou dermo-pharmaceutique, à base d'au moins un hydrocolloïde, et d'une structure de conditionnement apte à le contenir de manière étanche. L'invention est tout particulièrement adaptée aux patchs à action notamment tenseur, traitante, rafraîchissante, ou décontractante. De tels patchs ont une action cosmétique et/ou pharmaceutique sur la peau, par mise en contact avec la peau d'au moins une substance active dispersée dans une matrice. L'action, cosmétique et/ou pharmaceutique peut être obtenue rapidement (quelques minutes), ou résulter d'une mise en contact plus longue (par exemple, une heure ou plus).

Typiquement de tels patchs sont obtenus par découpe à la forme et aux dimensions voulues, d'une feuille constituée d'un support sur lequel est déposée puis séchée, une composition contenant un ou plusieurs actifs. Une telle réalisation fait intervenir des machines de couchage complexes, et génère des pertes importantes de matières premières, aussi bien en ce qui concerne le support que la composition contenant les actifs. De telles pertes sont d'autant plus importantes que la forme du patch n'est pas "simple" de manière à rendre ces patchs adaptés aux formes diverses et variées des différentes parties du visage, telles que le nez, le coin de l'oeil, le front, etc..

Enfin, une fois découpés, ces patchs doivent être conditionnés dans une pochette étanche, ce qui nécessite des manipulations supplémentaires, et donc augmente le coût de revient du patch.

Le document DE-A1-44 46 380 décrit une structure formée d'une barquette dans laquelle est déposée une couche d'hydrogel contenant un actif cosmétique ou thérapeutique. Une telle structure pose des problèmes, notamment liés au démoulage de la couche d'hydrogel en vue de son utilisation.

Aussi, est-ce une des objets de l'invention que de réaliser un patch, facile à démouler, et formé directement in situ, dans son conditionnement.

C'est un autre objet de l'invention que de réaliser un patch simple et économique à réaliser.

C'est encore un autre objet de l'invention que de pouvoir fournir un patch, apte, au contact de la peau, à provoquer des sensations nouvelles, notamment en termes de fraîcheur, de douceur, et de confort.

C'est également un autre objet de l'invention que de fournir un nouveau procédé de réalisation d'un patch, simple et économique à mettre en oeuvre.

D'autres objets apparaîtront de manière détaillée dans la description qui suit.

Ces objets sont atteints en réalisant un ensemble comportant une coque définissant au moins un compartiment présentant un fond et dont un bord délimite, dans une face opposée audit fond, une première ouverture, obturée par un premier élément de fermeture amovible, ledit compartiment contenant de manière amovible, un patch à la forme du compartiment, et constitué d'une matrice gélifiée obtenue par coulage dans ledit compartiment d'une composition contenant au moins un hydrocolloïde dans une phase aqueuse et au moins un composé actif, ladite matrice gélifiée comportant une trame, notamment sous forme d'un filet, d'un tissé ou d'un non tissé ou d'un film perforé.

Le patch obtenu peut contenir une grande quantité d'eau, et est donc frais à l'application, tout en donnant une grande impression de douceur. Le patch peut être appliqué directement sur la peau, sans mouillage préalable de celle-ci. Le patch est formé par moulage d'une composition dans une structure pouvant être utilisée comme conditionnement final du patch, c'est à dire comme conditionnement dans lequel est commercialisé le patch. Les opérations et manipulations nécessaires à la fabrication de ce patch sont donc réduites par rapport aux patchs de la technique antérieure. En outre, le patch réalisé peut être de forme quelconque, en fonction de l'application souhaitée. Il ne requiert aucune découpe, et par conséquent, sa réalisation ne génère aucune perte de matière.

La trame peut être sous forme d'un filet, notamment de polyamide. La présence de la trame présente plusieurs avantages. En premier lieu, elle permet de faciliter le démoulage du patch en vue de son utilisation, en particulier, mais pas exclusivement, lorsqu'elle est située au voisinage du fond du compartiment. En outre, elle donne de la tenue à la matrice, laquelle, lors de l'application ne se déforme pas, notamment par allongement. Ainsi, la trame autorise la réalisation de patchs de plus faible épaisseur. Par ailleurs, la trame permet de jouer sur la flexibilité du patch, et donc de permettre au patch de suivre parfaitement le profil de la surface sur laquelle il est appliqué. Enfin, le patch peut être réutilisé.

On entend par "hydrocolloïde" une macromolécule soluble dans l'eau, ne modifiant pas la valeur de l'activité en eau de la composition le contenant.

De préférence, la trame est déposée dans le fond du compartiment, préalablement au coulage de la composition, de sorte que la trame soit située au voisinage d'une face du patch adjacente au fond. Ainsi positionnée par rapport au compartiment, la trame joue son rôle d'assistance au démoulage en vue de l'utilisation du patch, en réduisant l'adhérence du patch au fond du compartiment.

Avantageusement, une partie de la trame n'est pas recouverte de ladite composition, de manière à former une zone de préhension en vue du démoulage du patch. Cette caractéristique est particulièrement précieuse lorsque la trame n'est pas située directement dans le fond du compartiment. En effet, en tirant sur cette portion de trame, non recouverte de ladite composition, on permet un démoulage facile du patch.

Le fond du compartiment peut être déformable, en réponse à une pression exercée par les doigts d'un utilisateur de manière à favoriser le démoulage du patch. Le compartiment peut être de profondeur non constante.

L'élément de fermeture amovible peut être constitué d'un opercule, notamment thermoscellé, d'une face d'une pochette contenant de manière hermétique ledit ensemble, ou du fond d'un autre ensemble empilé sur ledit ensemble. On peut en effet prévoir de commercialiser l'ensemble selon l'invention sous forme d'un empilement de plusieurs barquettes gerbables, lequel empilement serait conditionné dans un sachet de protection hermétique.

Le ou les hydrocolloïdes utilisés, peuvent être par exemple choisis dans le groupe formé par :
- la gomme de gellane ;
- la cellulose ou ses dérivés comme la carboxyméthylcellulose, l'hydroxypropyl-cellulose, la méthylcellulose, l'hydroxypropylméthylcellulose ou l'hydroxyéthyl-cellulose ainsi que les celluloses modifiées notamment par greffage de groupement alkyle ;
- les extraits d'algue tels que l'agar-agar, les carraghénanes, les alginates ;
- les extraits de graines tels que la gomme de caroube, la gomme de guar, les gommes de guar modifiées notamment par greffage de groupement alkyle ;
- les exsudats de plantes tels que la gomme arabique, la gomme karaya, la gomme adragante, la gomme de gatty ;
- les exsudats de micro-organismes tels que la gomme de xanthane ;
- les extraits de fruits tels que les pectines ;
- les agents gélifiants d'origine animale tels que la gélatine, les caséïnates ;
- les polymères synthétiques gélifiants hydrosolubles tels que les acides polyacryliques réticulés, éventuellement par une chaîne alkyle, tels que les "Carbopol" ou " Pemulen" de la Société GOODRICH ;
- les dérivés du silicium tels que les hectorites synthétiques comme les produits "Laponite RD et RDS" vendus par la société WAVERLY, les silicates d'aluminium et de magnésium comme le produit "Veegum" vendu par la société VANDERBILT,
- les produits du type POLYCARE®, commercialisé par la société Rhone-Poulenc.
ou un mélange de ces composés.

Avantageusement, on utilise en association avec la gomme de gellane, un hydrocolloïde choisi parmi la gomme de caroube, la gomme de xanthane, les dérivés de cellulose, une gomme de guar modifiée et les mélanges de ces composés. Il s'agit plus particulièrement de la gomme de xanthane, de la carboxyméthylcellulose ou d'une gomme de guar modifiée. Cette dernière peut être notamment l'hydroxypropylguar.

La composition contient de préférence au moins 0,5 % en poids de gomme de gellane, et au moins un deuxième hydrocolloïde choisi dans le groupe formé par la gomme de xanthane, les dérivés de cellulose, la gomme de caroube, une gomme de guar modifiée et leurs mélanges.

Avantageusement, la gomme de gellane est présente en une quantité allant de 0,5 à 15 % et de préférence de 0,5 à 6 % du poids total de la composition.

L'hydrocolloïde associé à la gomme de gellane est présent avantageusement en une quantité allant de 0,1 à 10 % et de préférence de 0,2 à 3 % du poids total de la composition.

De préférence, la phase aqueuse représente 60 à 97% du poids total de la composition, et de préférence 80 à 95% du poids total de la composition.

La composition peut contenir un solvant choisi parmi les alcools primaires tels que l'éthanol et l'isopropanol, les glycols tels que le propylène glycol, le butylène glycol, le dipropylène glycol, le diéthylène glycol, les éthers de glycol tel que les alkyl(C₁-C₄)éther de mono, di- ou tripropylène glycol, mono, di- ou triéthylène glycol, et leurs mélanges. L'alcool permet notamment d'apporter de la dureté et de la cohésion au gel.

La composition contient au moins un actif ayant un effet sur la peau, et pouvant être choisi notamment parmi les agents anti-oxydants, les anti-radicaux libres, les hydratants, les dépigmentants, les liporégulateurs, les anti-acnéiques, les anti-séborrhéiques, les agents anti-vieillissement, les adoucissants, les antirides, les kératolitiques, les anti-inflammatoires, les rafraîchissants, les cicatrisants, les protecteurs vasculaires, les anti-bactériens, les anti-fongiques, les anti-perspirants, les déodorants, les conditionneurs de la peau, les insensibilisants, les immunomodulateurs et nourrissants, ou les absorbeurs d'humidité (coton, polyacrylate), de sébum (Orgasol). La composition contient également de manière avantageuse, des conservateurs.

A titre d'exemples, la composition comporte au moins un actif hydrosoluble choisi parmi les composés suivants : l'acide ascorbique et ses sels biologiquement compatibles, les enzymes, des antibiotiques, les composants à effet tenseur, les α-hydroxy acides et leurs sels, les polyacides hydroxylés, les sucroses et leurs dérivés, l'urée, les aminoacides, les oligopeptides, les extraits végétaux hydrosolubles, et de levures, les hydrolysats de protéines, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone.

A titre d'exemples encore, la composition comporte au moins un composé liposoluble choisi parmi les composés suivants : D-α-tocophérol, DL-α-tocophérol, acétate de D-α-tocophérol, acétate de DL-α-tocophérol, palmitate d'ascorbyle, vitamine F et glycérides de vitamine F, vitamines D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches acides gras essentiels ; les kératolytiques tels que l'acide salicylique, ses sels et ses esters, l'acide n-octanoyl-5 salicylique et ses esters, alkylesters d'α-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, sphingomycline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

De préférence, la composition contient au moins un composé retardateur de prise, notamment sous forme d'un ou plusieurs sels. Le temps de "prise" du gel peut aller ainsi de 10 secondes à 24 heures.

Selon un mode de réalisation particulier, la matrice est colorée de manière à permettre la visualisation des impuretés ou résidus prélevés sur la peau lors de l'application et/ou l'enlèvement du patch. Cette caractéristique est particulièrement avantageuse pour les patchs à action nettoyante, contenant par exemple des absorbeurs de sébum, dans la mesure où elle permet de renseigner l'utilisatrice sur la fréquence et la nature du traitement à réaliser.

La matrice peut être colorée par incorporation de pigments synthétiques, minéraux, ou organiques. Les pigments colorés peuvent être constitués notamment de pigments du type de ceux utilisés dans le domaine de l'alimentaire ou de la cosmétique, en particulier pour les rouges à lèvres ou les vernis à ongles. A titre d'exemple, on peut citer, seuls ou en combinaison, des pigments synthétiques, ou des pigments minéraux, notamment des pigments d'oxyde de zirconium ou de cérium, ainsi que les oxydes de fer ou de chrome, et le bleu ferrique. On peut utiliser des pigments organiques, notamment le noir de carbone, les laques de baryum, strontium, calcium (DC Red N°7), aluminium. A titre d'exemple plus spécifique, on utilise un pigment portant la référence DC violet 2 K7014, commercialisé par KOHNSTAMM®.

La coque peut être formée par thermoformage ou injection à parois fines. On utilise de préférence un matériau ou un complexe de matériaux, étanche à l'oxygène et à la vapeur d'eau. Un tel matériau doit être choisi de manière à pouvoir résister à des températures de coulage qui peuvent atteindre 100 °C.

A titre préférentiel, la coque est formée d'un matériau thermoplastique choisi parmi les polyéthylènes, ou les polypropylènes, ou d'un complexe de matériaux thermoplastiques, tel que le Polyéthylène Téréphtalate/Aluminium/Polyéthylène, ou les élastomères, notamment les élastomères de silicone, ou élastomères thermoplastiques. La réalisation de la coque en un matériau élastiquement déformable permet de faciliter le démoulage du patch, notamment en vue de son utilisation.

Selon une première forme de réalisation de l'ensemble selon l'invention, la matrice est moulée dans ledit compartiment, par coulage via ladite première ouverture, de ladite composition.

Avantageusement, la coque forme un rebord tout autour de ladite première ouverture, de manière à y permettre la fixation, notamment par collage, thermoscellage ou soudure, du premier élément de fermeture.

Le premier élément de fermeture peut être constitué d'un opercule, formé d'un matériau métallique (Aluminium) ou thermoplastique tel qu'un polyéthylène, un polypropylène, d'une céramique, ou d'un complexe de tels matériaux. On assure ainsi une parfaite conservation du patch ainsi conditionné. L'enlèvement d'un tel opercule peut être facilité par la présence d'un "coin cassé" formé par la coque, et auquel est collé l'opercule, de manière à en faciliter l'enlèvement par arrachage. On choisira de préférence, des matériaux peu adhérant sur la barquette, et des cordons de soudure pourront être aménagés afin d'assurer une bonne fermeture.

Alternativement, la composition est coulée dans le compartiment par le fond. Dans cette hypothèse, le compartiment comprend au moins une seconde ouverture, ménagée dans ledit fond, ladite matrice étant moulée dans le compartiment, par coulage via ladite seconde ouverture, de ladite composition, ladite seconde ouverture étant obturée après remplissage du compartiment. L'élément de fermeture amovible obturant la première ouverture, est, dans ce cas de figure, mis en place avant coulage de la composition par le fond.

La seconde ouverture peut être obturée par un opercule, collé ou soudé autour de ladite seconde ouverture.

La trame peut être disposée au voisinage d'une première face du patch opposée au fond du compartiment. Tel peut être le cas lorsque la composition est coulée notamment par le fond. Alternativement, la trame est située, au voisinage d'une seconde face du patch adjacente au fond du compartiment. Dans ce cas, la composition est coulée par le dessus, en ayant, préalablement au coulage, déposé ladite trame dans le fond du compartiment. Alternativement encore, la trame est noyée dans la matrice, dans une position située entre la première et la seconde face. A cet effet, on peut couler la composition par le dessus. On forme alors une première couche de ladite composition dans le fond du compartiment. On dépose la trame sur la première couche, et on coule ensuite une deuxième couche de ladite composition.

Selon un autre aspect de l'invention, on réalise également un procédé de réalisation d'un patch cosmétique ou pharmaceutique consistant :
a) dans une coque définissant au moins un compartiment, couler une composition contenant au moins un hydrocolloïde dans une phase aqueuse, et au moins un composé actif, une trame, notamment sous forme d'un filet, d'un tissé ou d'un non tissé, ou d'un film perforé, étant introduite dans le compartiment; et
b) laisser figer la composition de manière à former un gel sous forme d'une feuille contenue de manière amovible à l'intérieur du compartiment, et apte à réaliser ledit patch cosmétique ou pharmaceutique à la forme dudit compartiment.

La composition peut être coulée dans le compartiment à une température voisine de 90 °C, le figeage ou gélification de la composition sous forme d'un gel se produisant aux environs de 60 à 70 °C. Dans la pratique, bien que ne constituant pas un mode préférentiel, la composition peut être coulée en cours de figeage.

Le fond du compartiment peut faire l'objet d'un traitement anti-adhérant, notamment sous forme d'un dépôt de glycérine ou d'une composition équivalente, préalablement au coulage de la composition (P).

L'invention consiste, mises à part les dispositions exposées ci-dessus, en un certain nombre d'autres dispositions qui seront explicitées ci-après, à propos d'exemples de réalisation non limitatifs, décrits en référence aux figures annexées, parmi lesquelles :
- la figure 1 représente un premier mode de réalisation du dispositif selon l'invention;
- les figures 2A-2C illustrent un premier mode de remplissage du dispositif de la figure 1;
- les figures 3A-3C illustrent des variantes du procédé des figures 2A-2C; et
- les figures 4A-4C illustrent un second mode de remplissage du dispositif de la figure 1.

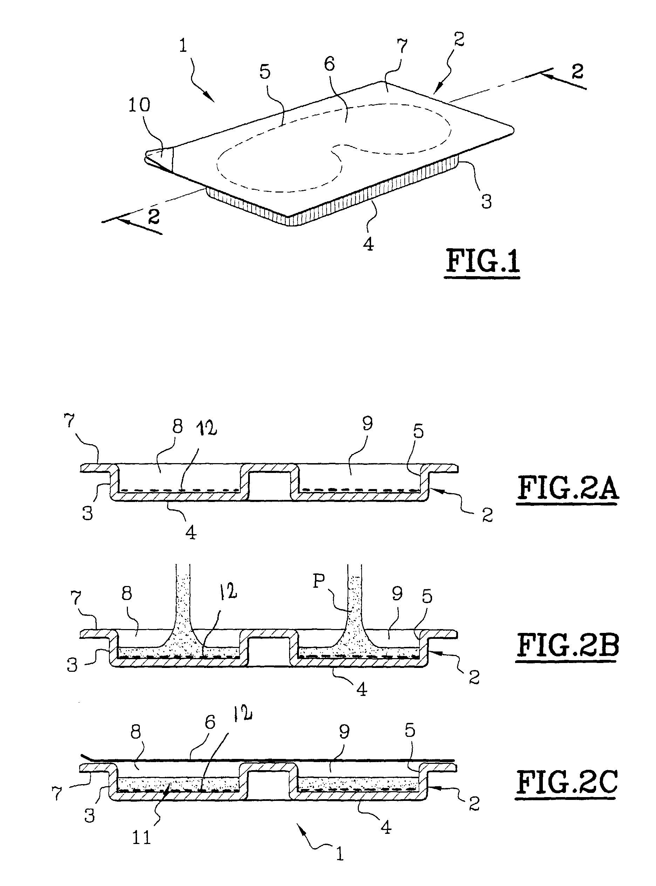

A la figure 1, l'ensemble 1 représenté comprend une coque 2 obtenue par thermoformage ou injection à parois fines d'un matériau tel qu'un polypropylène. La coque 2 forme un compartiment en creux 3 dont la forme correspond à celle d'un masque pour les yeux. Ledit compartiment présente un bord délimitant sur sa face opposée au fond 4, une ouverture 5, obturée par un opercule thermoscellé 6. L'opercule thermoscellé 6 est formé d'un complexe Polypropylène/Aluminium/Polyéthylène. A cet effet, la coque forme un rebord 7 tout autour de l'ouverture 5, de manière à permettre la fixation de l'opercule 6, et ce, de manière étanche tout autour de l'ouverture 5. La coque 2 présente un coin cassable 10, permettant de favoriser l'enlèvement de l'opercule 6, en vue de l'utilisation d'un patch cosmétique contenu à l'intérieur du compartiment 3, ledit patch étant, comme on le verra plus en détail par la suite, formé directement in-situ, à l'intérieur du compartiment 3.

Les figures 2A-2C illustrent une vue en coupe selon la ligne 2A-2C de l'ensemble 1 de la figure 1. A la figure 2A, la coque 2 est posée sur son fond, l'ouverture 5 n'étant pas obturée. Une trame 12, sous forme d'un filet de polyamide, d'une épaisseur de 0,2 mmm, est déposée dans le fond 4 du compartiment 3. La trame est sensiblement à la forme et aux dimensions du compartiment. A la figure 2B, la composition liquide P apte à former la matrice gélifiée du patch est coulée sur la trame 12, au moyen de deux becs situés en regard de chacune des deux zones 8, et 9 du compartiment 3. Dans cette phase de coulage, la température de la composition liquide P est de l'ordre de 90 °.

Lors du refroidissement de la composition P, la composition liquide se fige ou gélifie de manière à former un patch gélifié 11 à la forme et aux dimensions (hormis la profondeur) du compartiment 3. Typiquement, le figeage ou gélification se produit à une température qui peut être de l'ordre de 60 °C à 70 °C. Avant, pendant, ou après le figeage de la composition, un opercule 6 est thermoscellé sur la coque 2, de manière à obturer l'ouverture 5. Le figeage résulte notamment de l'abaissement de la température, ou d'une réaction avec l'eau contenue dans la matrice. Une fois la composition figée, la trame 12 est prisonnière de la matrice, et se situe au voisinage du fond 4, où elle forme des "reliefs", aptes à réduire l'adhérance du patch gélifié 11 dans le fond du compartiment. Le démoulage s'en trouve ainsi favorisé.

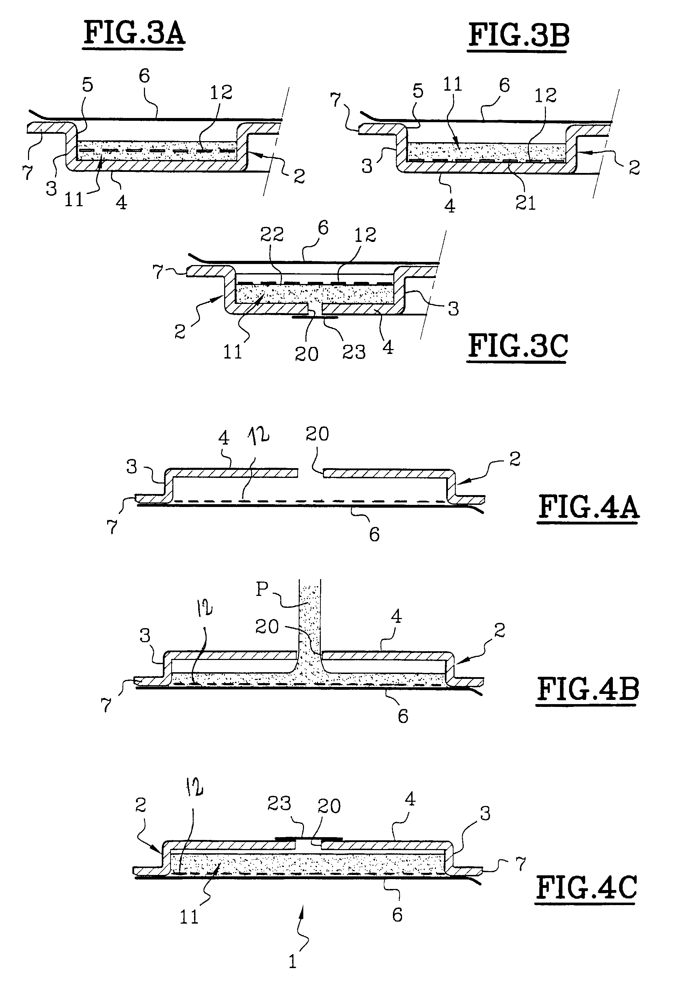

Les figures 3A-3C illustrent des variantes du mode de réalisation précédent. A la figure 3A, une trame 12, formée d'un filet de polyamide, est noyée dans la matrice du patch 11. Pour ce faire, on a procédé au coulage de la composition, au travers de l'ouverture 5, et ce en deux étapes successives. Dans une première étape, une première couche de ladite composition P est déposée dans le fond du compartiment 3. La trame 12 est ensuite déposée à la surface de cette première couche. Une seconde couche de ladite composition est ensuite déposée sur la trame 12. La trame permet de réduire l'épaisseur du patch tout en conservant une intégrité suffisante. L'épaisseur du patch peut aller de quelques 10èmes de mm à quelques mm, en fonction de l'application souhaitée. La trame peut avoir une épaisseur comprise entre 0, 01 mm et 2 mm. Son poids peut aller de 5 à 60 g/m², et de préférence, de 10 à 40 g/m². Bien que non représenté, il est possible de prévoir une petite portion de trame, notamment une zone de bord, qui ne soit pas recouverte de composition, de manière à former une zone de préhension en vue du démoulage du patch 11.

Dans le mode de réalisation de la figure 3B, la trame 12 est déposée dans le fond 4 du compartiment 3 avant le coulage de la composition de sorte que ladite trame soit située au voisinage de la face 21 du patch 11 adjacente au fond 4. Ce mode de réalisation correspond à celui des figures 2A-2C.

Dans le mode de réalisation de la figure 3C, le coulage de la composition est effectué, comme on le verra plus en détail par la suite, via une ouverture 20, ménagée dans le fond 4 du compartiment 3. L'opercule 6 est thermoscellé sur l'ouverture 5 avant le coulage de la composition, et de ce fait, fait office de fond pour le coulage de la composition. Avant coulage, une trame 12 est déposée à l'intérieur du compartiment. Dans la pratique, la trame est disposée dans le compartiment 3, avant scellage de l'ouverture 5. En retournant l'ensemble, la trame 12 vient se positionner en contact de l'opercule 6. Ainsi, dans le patch final, la trame 12 est située au voisinage d'une face 22 du patch 11 située en regard de l'opercule thermoscellé 6. Après coulage de la composition, et de préférence, avant figeage, l'ouverture 20 est obturée par un opercule 23 collé ou thermoscellé. L'ensemble 1 est ensuite retourné de sorte que le figeage ou gélification de la matrice se fasse en contact du fond 4 du compartiment 3. La trame 12 migre légèrement en direction du fond 4 du compartiment, de manière à se noyer dans la matrice, et à jouer pleinement son rôle.

Les figures 4A-4C illustrent une variante du mode de remplissage des figures 2A-2C, et selon laquelle la composition est coulée dans le compartiment 3 via une ouverture 20 ménagée dans le fond 4 du compartiment. A la figure 4A, le compartiment 3, dont l'ouverture 5 a été préalablement scellée par l'opercule 6, est retourné tête en bas. Avant scellage de l'opercule 6, une trame 12 est déposée dans le compartiment 3. A la figure 4B, la composition P est coulée dans le compartiment 3, via l'ouverture 20 ménagée dans le fond 4 du compartiment 3. Une seconde ouverture (non représentée) peut être ménagée dans le fond du compartiment de manière à permettre l'échappement d'air lors du coulage à chaud. A la figure 4C, l'ouverture 20 est scellée au moyen d'une pastille adhésive 23, collée ou thermoscellée. De préférence, l'ensemble 1 est retourné avant figeage ou gélification de la composition liquide P. Le remplissage de la barquette peut être complet ou partiel. Dans cette configuration, la trame 12 sert essentiellement à conférer de la tenue au patch, et à éviter notamment qu'il se déforme par allongement lors de son utilisation.

Pour utiliser le patch selon l'invention, l'utilisatrice enlève l'opercule 6. En provoquant une légère flexion du fond 4 du compartiment 3, elle démoule le patch 11, et le prend avec les doigts pour l'appliquer sur la partie correspondante du visage ou du corps. A cet effet, le patch peut être de formes diverses. Il peut être notamment adapté à la forme des yeux, du front, du nez, du tour de la bouche, des joues, ou de toute autre partie du corps. Le démoulage du patch 11 peut être favorisé en réalisant la barquette 2 en matériau élastiquement déformable, notamment en élastomère thermoplastique.

### EXEMPLE 1 :

Dans 180 g d'eau, on incorpore :
- 3% en poids de gomme de gellane;
- 1% en poids de gomme de xanthane;
- 1% de germe de blé;
- 0,2% de conservateur
- 2% d'orgasol; et
- 0,15% d'huile essentielle de lavande.

La composition est coulée à une température de 90° dans une barquette thermoformée en polypropylène. La composition est coulée dans le fond de la barquette sur une épaisseur de 1 mm, un filet de polyamide étant préalablement déposé dans le fond de la barquette. Après coulage, la barquette est refermée par un opercule thermoscellé.

Après démoulage par l'utilisatrice, le patch est appliqué sur le visage pendant 5 mn à 60 mn. Un tel patch a une action décontractante, et tenseur apaisante.

### EXEMPLE 2 :

Dans 180 g d'eau, on incorpore :
- 2,5% en poids de gomme de gellane;
- 0,7% en poids de gomme de xanthane;
- 1% d'alginate;
- 0,5% d'acide ascorbique;
- 0,5% de conservateur;
- 2% d'éthanol; et
- 2% de SPF.

La composition est coulée à une température de 90° dans une barquette injectée en Polyéthylène. La composition est coulée dans le fond de la barquette sur une épaisseur de 1 mm, un non tissé étant préalablement déposé dans le fond de la barquette. Après coulage, la barquette est refermée par un opercule thermoscellé.

Après démoulage par l'utilisatrice, le patch est appliqué sur le visage pendant 5 mn à 60 mn. Un tel patch a une action apaisante, éclaircissante, et nivelante.

### EXEMPLE 3 :

Dans 180 g d'eau, on incorpore :
- 2,5% en poids de gomme de gellane;
- 0,7% en poids de gomme de xanthane;
- 2% d'extrait de fleur;
- 0,5% d'éthanol; et
- 0,5% de cristaux de menthe.

La composition est coulée à chaud, à une température de 90° dans une barquette injectée en Polyéthylène. La composition est coulée dans le fond de la barquette sur une épaisseur de 1 mm, un filet de polyamide étant préalablement déposé dans le fond de la barquette. Après coulage, la barquette est refermée par un opercule thermoscellé.

Après démoulage par l'utilisatrice, le patch est appliqué sur le visage pendant 5 mn à 60 mn. Un tel patch a une action rafraîchissante et aseptisante.

Dans la description détaillée qui précède, il a été fait référence à des modes de réalisation préférés de l'invention. Il est évident que des variantes peuvent y être apportées sans s'écarter de l'esprit de l'invention telle que revendiquée ci-après.

## Revendications

1. Ensemble (1) comportant une coque (2) définissant au moins un compartiment (3) présentant un fond (4) et dont un bord délimite, dans une face opposée audit fond, une première ouverture (5), obturée par un premier élément de fermeture amovible (6), ledit compartiment (3) contenant de manière amovible un patch (11) à la forme du compartiment, et constitué d'une matrice gélifiée obtenue par coulage dans ledit compartiment (3) d'une composition (P) contenant au moins un hydrocolloïde dans une phase aqueuse et au moins un composé actif, ladite matrice gélifiée comportant une trame (12), notamment sous forme d'un filet, d'un tissé ou d'un non tissé ou d'un film perforé.

2. Ensemble selon la revendication 1 caractérisé en ce que la trame (12) est déposée dans le fond (4) du compartiment (3), préalablement au coulage de la composition (P), de sorte que la trame (12) soit située au voisinage d'une face (21) du patch, adjacente au fond (4).

3. Ensemble selon la revendication 1 ou 2 caractérisé en ce qu'une partie de la trame (12) n'est pas recouverte de ladite composition (P), de manière à former une zone de préhension en vue du démoulage du patch (11).

4. Ensemble selon l'une quelconque des revendications 1 à 3 caractérisé en ce que le fond (4) du compartiment (3) est déformable, en réponse à une pression exercée par les doigts d'un utilisateur de manière à favoriser le démoulage du patch (11).

5. Ensemble selon l'une quelconque des revendications 1 à 4 caractérisé en ce que le compartiment (3) est de profondeur non constante.

6. Ensemble selon l'une quelconque des revendications 1 à 5 caractérisé en ce que ledit élément de fermeture amovible est constitué d'un opercule, notamment thermoscellé (6), d'une face d'une pochette contenant de manière hermétique ledit ensemble (1), ou du fond (4) d'un autre ensemble empilé sur ledit ensemble (1).

7. Ensemble selon l'une quelconque des revendications 1 à 6 caractérisé en ce que le (ou les) hydrocolloïde(s) est (sont) choisi(s) parmi la gomme de gellane, la cellulose ou ses dérivés ; les extraits d'algues ; les extraits de graines ; les exsudats de plantes ; les exsudats de micro-organismes telle que la gomme de xanthane ; les extraits de fruits ; les agents gélifiants d'origine animale ; les polymères synthétiques gélifiants hydrosolubles; les dérivés du silicium ; et leurs mélanges.

8. Ensemble selon la revendication 7 caractérisé en ce que la composition (P) contient au moins 0,5 % en poids de gomme de gellane, et au moins un deuxième hydrocolloïde choisi dans le groupe formé par la gomme de xanthane, les dérivés de cellulose, la gomme de caroube, une gomme de guar modifiée et leurs mélanges.

9. Ensemble selon la revendication précédente, caractérisé en ce que la gomme de gellane est présente en une quantité allant de 0,5 à 15 % et de préférence de 0,5 à 6 % du poids total de la composition (P).

10. Ensemble selon la revendication 8 ou 9, caractérisé en ce que l'hydrocolloïde associé à la gomme de gellane est présent en une quantité allant de 0,1 à 10 % et de préférence de 0,2 à 3 % du poids total de la composition (P).

11. Ensemble selon l'une quelconque des revendications 1 à 10 caractérisé en ce que la phase aqueuse représente 60 à 97% du poids total de la composition (P).

12. Ensemble selon l'une quelconque des revendications précédentes caractérisé en ce que la composition (P) contient un solvant choisi parmi les alcools primaires tels que l'éthanol et l'isopropanol, les glycols tels que le propylène glycol, le butylène glycol, le dipropylène glycol, le diéthylène glycol, les éthers de glycol tel que les alkyl(C₁-C₄)éther de mono, di- ou tripropylène glycol, mono, di- ou triéthylène glycol, et leurs mélanges.

13. Ensemble selon l'une quelconque des revendications précédentes, caractérisé en ce que la composition (P) contient au moins un actif ayant un effet sur la peau, choisi notamment parmi les agents anti-oxydants, les anti-radicaux libres, les hydratants, les dépigmentants, les liporégulateurs, les anti-acnéiques, les anti-séborrhéiques, les agents anti-vieillissement, les adoucissants, les anti-rides, les kératolitiques, les anti-inflammatoires, les rafraîchissants, les cicatrisants, les protecteurs vasculaires, les anti-bactériens, les anti-fongiques, les anti-perspirants, les déodorants, les conditionneurs de la peau, les insensibilisants, les immunomodulateurs et nourrissants, ou les absorbeurs d'humidité (coton, polyacrylate), de sébum (Orgasol).

14. Ensemble selon la revendication 13 caractérisé en ce que la composition (P) comporte au moins un actif hydrosoluble choisi parmi les composés suivants : l'acide ascorbique et ses sels biologiquement compatibles, les enzymes, des antibiotiques, les composants à effet tenseur, les α-hydroxy acides et leurs sels, les polyacides hydroxylés, les sucroses et leurs dérivés, l'urée, les aminoacides, les oligopeptides, les extraits végétaux hydrosolubles, et de levures, les hydrolysats de protéines, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone.

15. Ensemble selon la revendication 13 caractérisé en ce que la composition (P) comporte au moins un composé liposoluble choisi parmi les composés suivants : D-α-tocophérol, DL-α-tocophérol, acétate de D-α-tocophérol, acétate de DL-α-tocophérol, palmitate d'ascorbyle, vitamine F et glycérides de vitamine F, vitamines D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches acides gras essentiels ; les kératolytiques tels que l'acide salicylique, ses sels et ses esters, l'acide n-octanoyl-5 salicylique et ses esters, alkylesters d'α-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, sphingomycline de lait, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

16. Ensemble selon l'une quelconque des revendications 1 à 15 caractérisé en ce que la composition (P) contient au moins un composé retardateur de prise, notamment sous forme d'au moins un sel.

17. Ensemble selon l'une quelconque des revendications précédentes caractérisé en ce que la matrice gélifiée est colorée de manière à permettre la visualisation des impuretés ou résidus prélevés sur la peau lors de l'application et/ou l'enlèvement du patch (11).

18. Ensemble selon la revendication 17 caractérisé en ce que la matrice est colorée par incorporation de pigments synthétiques, minéraux, ou organiques.

19. Ensemble selon l'une quelconque des revendications 1 à 18 caractérisé en ce que la coque (2) est formée par thermoformage ou injection à parois fines.

20. Ensemble selon la revendication 19 caractérisé en ce que la coque (2) est formée d'un matériau thermoplastique choisi parmi les polyéthylènes, ou les polypropylènes, ou d'un complexe de matériaux thermoplastiques, tel que le Polyéthylène Téréphtalate/Aluminium/Polyéthylène, ou les élastomères, notamment les élastomères de silicone.

21. Ensemble selon l'une quelconque des revendications 1 à 20 caractérisé en ce que la matrice est moulée dans ledit compartiment (3), par coulage via ladite première ouverture (5), de ladite composition (P).

22. Ensemble selon l'une quelconque des revendications 1 à 21 caractérisé en ce que la coque (2) forme un rebord (7) tout autour de ladite première ouverture (5), de manière à y permettre la fixation, notamment par collage, thermoscellage ou soudure, du premier élément de fermeture (6).

23. Ensemble selon l'une quelconque des revendications 1 à 22 caractérisé en ce que le premier élément de fermeture (6) est constitué d'un opercule, formé d'un matériau métallique ou thermoplastique tel qu'un polyéthylène, un polypropylène, ou d'un complexe de tels matériaux.

24. Ensemble selon l'une quelconque des revendications 1 à 20 caractérisé en ce qu'il comprend au moins une seconde ouverture (20), ménagée dans ledit fond (4), ladite matrice étant moulée dans le compartiment (3), par coulage de ladite composition (P) via ladite seconde ouverture (20), ladite seconde ouverture (20) étant obturée après remplissage du compartiment.

25. Ensemble selon la revendication 24 caractérisé en ce que ladite seconde ouverture (20) est obturée par un opercule (23), collé ou soudé autour de ladite seconde ouverture.

26. Procédé de réalisation d'un patch cosmétique ou pharmaceutique (11) consistant :
a) dans une coque (2) définissant au moins un compartiment (3), couler une composition (P) contenant au moins un hydrocolloïde dans une phase aqueuse, et au moins un composé actif, une trame (12), notamment sous forme d'un filet, d'un tissé ou d'un non tissé, ou d'un film perforé, étant introduite dans le compartiment ; et
b) laisser figer la composition (P) de manière à former une matrice gélifiée sous forme d'une feuille contenue de manière amovible à l'intérieur du compartiment (3), et apte à réaliser ledit patch cosmétique ou pharmaceutique (11) à la forme dudit compartiment (3).

27. Procédé selon la revendication 26 caractérisé en ce que la composition (P) est coulée au travers d'une première ouverture (5) formée par un bord du compartiment (3), et située à l'opposée d'un fond (4) dudit compartiment, la trame (12) étant déposée dans le fond (4) du compartiment (3), avant le coulage de la composition (P).

28. Procédé selon la revendication 27 caractérisé en ce que ladite première ouverture (5) est obturée de manière amovible, notamment par un opercule (6).

29. Procédé selon la revendication 26 caractérisé en ce que la composition (P) est coulée dans le compartiment (3) via une seconde ouverture (20) ménagée dans un fond (4) du compartiment (3).

30. Procédé selon l'une quelconque des revendications 26 à 29 caractérisé en ce que la composition (P) est coulée dans le compartiment (3) à une température voisine de 90 °C.

31. Procédé selon l'une quelconque des revendications 26 à 30 caractérisé en ce que le fond (4) du compartiment (3) fait l'objet d'un traitement anti-adhérant, préalablement au coulage de la composition (P).
